# EUROPEAN PATENT APPLICATION

(11) **EP 3 431 071 A1**
(43) Date of publication of application: **23.01.2019**
(21) Application number: 17382473.1
(22) Date of filing: 18.07.2017
(51) Int. Cl.: A61K 8/362, A61K 8/41, A61Q 5/08

(54) **ANHYDROUS COMPOSITION FOR BLEACHING KERATIN FIBRES**

(71) Applicant: Revlon, New York, NY 10004 (US)
(72) Inventor: Garcés Gómez de Aranda, Carlos, 08018 Barcelona (ES); Vallecillos López, Rocio, 08018 Barcelona (ES)
(74) Representative: Gallardo, Antonio M.

(57) **Abstract**

Anhydrous compositions for bleaching keratin fibers are described. Said compositions comprise at least one cationic polymer and maleic acid and/or a cosmetically acceptable salt thereof.

A process for bleaching keratin fibers using these compositions and a packaging kit containing these compositions are also described.

## Description

### TECHNICAL FIELD

Anhydrous compositions for bleaching keratin fibers are described. Said compositions comprise at least one cationic polymer and maleic acid and/or a cosmetically acceptable salt thereof.

### BACKGROUND

The information provided below is not admitted to be prior art to the present disclosure, but is provided solely to assist the understanding of the reader.

Keratin fibers, such as human hair, can be bleached (i.e. highlighted or decolored) by oxidizing the melanin pigment in the hair. The result of oxidizing the melanin pigment is a dissolution and partial or total removal of this pigment. Bleaching powders containing a peroxygenated reagent such as ammonium or alkali metal persulfates, perborates and percarbonates are combined with an aqueous hydrogen peroxide at the time of use to achieve melanin oxidization.
It is known that bleaching powders and hydrogen peroxide combinations are chemically active on hair and damage hair, especially after repeated bleaching processes. Damaged hair does not behave as natural hair does and requires the use of special conditioning and/or daily care products to keep hair looking attractive. This is time consuming and expensive and, therefore, there is a need for a simple to apply new bleaching composition that causes a reduced degree of hair damage.

### SUMMARY

Described herein is an anhydrous composition for bleaching keratin fibers comprising at least one cationic polymer and maleic acid and/or a cosmetically acceptable salt thereof.

In an embodiment disclosed herein the at least one cationic polymer is selected from Polyquaternium-6, Polyquaternium-7, Polyquaternium-10, Polyquaternium-16, Polyquaternium-22 and mixtures thereof.

In an embodiment disclosed herein the total amount of the at least one cationic polymer is in the range of about 0.01 to about 10 wt. %, based on the total weight of the composition.

In an embodiment disclosed herein the maleic acid is present in acid form.

In an embodiment disclosed herein the total amount of maleic acid and/or a cosmetically acceptable salt thereof is in the range of about 0.01 to about 10 wt. %, based on the total weight of the composition.

In an embodiment disclosed herein is composition for bleaching human keratin fibers as defined above, where the composition further comprises anionic, nonionic, cationic, amphoteric or zwitterionic surfactants and mixtures thereof, petroleum hydrocarbons (mineral oils, paraffins and waxes), vegetable fats and oils, fatty acids, fatty alcohols, natural waxes, silicones, granulating adjuvants, binders, mineral fillers, lubricants, opacifiers, inorganic pigments, dyes, sequestering agents, fragrances or perfumes and nonionic or amphoteric polymers.

In an embodiment disclosed herein is a process for bleaching keratin fibers comprising the steps of mixing, before application, an anhydrous composition comprising at least one cationic polymer and maleic acid and/or a cosmetically acceptable salt thereof with an aqueous hydrogen peroxide composition; topically applying the mixture obtained to the keratin fibers to be bleached; leaving the mixture to stand on the fibers for a period which is sufficient to obtain the desired bleaching effect; and removing the mixture from the keratin fibers.

In an embodiment disclosed herein is a process for bleaching keratin fibers as defined above further comprising the step of topically applying to the keratin fibers a composition comprising at least one conditioning agent, then rinsing with water and optionally followed by washing the keratin fibers with a shampoo, rinsing with water, and optionally drying; or washing the keratin fibers with a shampoo, then rinsing with water and optionally followed by topically applying to the keratin fibers a composition comprising at least one conditioning agent, rinsing with water, and optionally drying.

In an embodiment disclosed herein is a multi-compartment device for bleaching keratin fibers comprising at least two compartments, wherein one compartment comprises a composition comprising at least one cationic polymer and maleic acid and/or a cosmetically acceptable salt thereof; and a second compartment comprising an aqueous hydrogen peroxide composition.

### DETAILED DESCRIPTION

Embodiments of the present disclosure are discussed in detail below. In describing embodiments, specific terminology is employed for the sake of clarity. However, the disclosure is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the disclosure. While a number of embodiments and features are described herein, it is to be understood that the various features of the disclosure and aspects of embodiments, even if described separately, may be combined unless mutually exclusive or contrary to the specific description. All references cited herein are incorporated by reference as if each had been individually incorporated.

The terms "about" or "approximately" as used herein shall generally mean within 20 percent, or within 10 percent of a given value. Other than in the operating examples, or where otherwise indicated, all numbers expressing quantities of ingredients and/or reaction conditions are to be understood as being modified in all instances by the term "about".

The term "anhydrous" as used herein refers to a composition whose water content is less than 1 % by weight, relative to the total weight of the composition.

The term "keratin fibers" as used herein refers to a family of proteins that occur in the vertebrates and exercise a protective function. In an embodiment, keratin fibers refers to human keratin fibers. In another embodiment, keratin fibers refers to human hair.

The damage of hair can be measured by its cysteic acid level since cysteic acid is formed due to hair fiber degradation. The higher the hair damage, the higher cysteic acid level in hair. Watanabe K et al, Cysteic Acid Formation Behaviors in Bleached Hair of Southeast Asian Characterized by Infrared Spectroscopy, Advances in Life Sciences, 5(4), 85-89 (2015), cited sections herein incorporated by reference.

Another way of measuring the damage level of hair is by hair breakage. The hair breakage is determined by using a multiple combing test. The higher the hair damage, the higher the amount of broken fibers. Haake H-M et al, Hair breakage - How to measure and counteract, J. Cosmet. Sci.,60, 143-151 (2009), cited sections herein incorporated by reference.

The damage level of hair can also be evaluated by visual inspection of hair in terms of feeling, shine, or healthy appearance.

Typically, an anhydrous composition for bleaching keratin fibers contain at least one alkaline agent and at least one peroxygenated salt.

### The alkaline agent

As indicated above, the anhydrous composition for bleaching keratin fibers may comprise at least one alkaline agent. An alkaline agent is a compound having a pH above 7. The addition of an alkaline agent to the anhydrous composition adjusts the pH of the composition to a less acid pH.

Typically, in the anhydrous composition for bleaching keratin fibers, the total amount of the at least one alkaline agent is in the range of about 1 to about 60 wt. %, or about 5 to about 50 wt. %, based on the total weight of the composition.

Examples of suitable alkaline agents include, but are not limited to, ammonium salts, alkali metal or alkaline-earth metal salts of silicates, metasilicates, phosphates, hydrogen phosphates, carbonates, hydrogen carbonates, and mixtures thereof.

The alkaline agents may include, but are not limited to, ammonium chloride, ammonium bromide, ammonium iodide, ammonium sulfate, ammonium nitrate, ammonium phosphates, ammonium nitrate, ammonium dihydrogen phosphate, ammonium hydrogen phosphate, diammonium sodium phosphate, sodium ammonium hydrogen phosphate, ammonium disodium phosphate, ammonium carbonate, ammonium hydrogen carbonate, sodium silicate, potassium silicate, sodium metasilicate, potassium metasilicate, sodium carbonate, potassium carbonate, magnesium carbonate, magnesium carbonate hydroxide (magnesium basic carbonate), and mixtures thereof. Typically, the alkaline agents are sodium silicate, potassium silicate, sodium metasilicate, potassium metasilicate, magnesium carbonate, magnesium carbonate hydroxyde, and mixtures thereof.

### The peroxygenated salt

As indicated above, the anhydrous composition for bleaching keratin fibers may comprise at least one peroxygenated salt.

Typically, in the anhydrous composition for bleaching keratin fibers, the total amount of the at least one peroxygenated salt is in the range of about 20 to about 70 wt. %, or in the range of about 25 to about 65 wt. %, based on the total weight of the composition.

Examples of suitable peroxygenated salts include, but are not limited to, ammonium, alkali metal or alkaline-earth metal persulfates, percarbonates, perborates, peroxides, and mixtures thereof.

Peroxygenated salts may be ammonium persulfate, sodium persulfate, potassium persulfate, magnesium peroxide, and mixtures thereof. Typically, peroxygenated salts are sodium persulfate, potassium persulfate, ammonium persulfate and mixtures thereof.

### The cationic polymer

The anhydrous composition for bleaching keratin fibers comprises at least one cationic polymer.

The term "cationic polymer" as used herein refers to a macromolecule (polymer) that contains at least one monomer bearing a quaternary ammonium group.

Typically, in the anhydrous composition for bleaching human keratin fibers of the disclosure, the total amount of the least one cationic polymer is in the range of about 0.01 to about 10 wt. %, based on the total weight of the composition.

In an embodiment, in the anhydrous composition for bleaching keratin fibers of the disclosure, the total amount of the least one cationic polymer is in the range of about 0.01 to about 7.5 wt. %, based on the total weight of the composition.

In another embodiment, in the anhydrous composition for bleaching keratin fibers of the disclosure, the total amount of the least one cationic polymer is in the range of about 0.05 to about 5 wt. %, based on the total weight of the composition.

Examples of suitable cationic polymers include, but are not limited to, those polymers known by their International Nomenclature for Cosmetic Ingredients (INCI) name as Polyquaternium. Typical examples of those are Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-52, Polyquaternium-53, Polyquaternium-54, Polyquaternium-55, Polyquaternium-56, Polyquaternium-57, Polyquaternium-58, Polyquaternium-59, Polyquaternium-60, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-70, Polyquaternium-71, Polyquaternium-72, Polyquaternium-73, Polyquaternium-74, Polyquaternium-75, Polyquaternium-76, Polyquaternium-77, Polyquaternium-78, Polyquaternium-79, Polyquaternium-80, Polyquaternium-81, Polyquaternium-82, Polyquaternium-83, Polyquaternium-84, Polyquaternium-85, Polyquaternium-86, Polyquaternium-87, Polyquaternium-88, Polyquaternium-91, Polyquaternium-92, Polyquaternium-94, Polyquaternium-95, Polyquaternium-96, Polyquaternium-98, Polyquaternium-99, Polyquaternium-100, Polyquaternium-102, Polyquaternium-104, Polyquaternium-109, Polyquaternium-110, Polyquaternium-111, Polyquaternium-112, Polyquaternium-113 and Polyquaternium-114. Other suitable cationic polymers include those known by their INCI name as Guar hydroxypropyl trimonium chloride, Polyacrylamidopropyltrimonium Chloride, Polymethacrylamidopropyltrimonium Chloride and Polymethacrylamidopropyltrimonium Methosulfate.

Typically, cationic polymers according to the invention include, but are not limited to, Polyquaternium-6, Polyquaternium-7, Polyquaternium-10, Polyquaternium-16, Polyquaternium-22 and mixtures thereof; or are Polyquaternium-7, Polyquaternium-10, Polyquaternium-22 and mixtures thereof.

An example of a suitable cationic polymer according to the present disclosure is Polyquaternium-6, a polymer of dimethyl diallyl ammonium chloride.

Another example of a suitable cationic polymer according to the present disclosure is Polyquaternium-7, a polymeric quaternary ammonium salt consisting of acrylamide and dimethyl diallyl ammonium chloride monomers.

Another example of a suitable cationic polymer according to the present disclosure is Polyquaternium-10. Polyquaternium-10, also known as Quaternium-19, is a polymeric quaternary ammonium salt of hydroxyethyl cellulose reacted with trimethyl ammonium substituted epoxide. As given in the Final Report on the Safety Assessment of Polyquaternium-10, J Am Coll Toxicol, Volume 7, Number 3 (1988), there are various grades of Polyquaternium-10 with different average molecular weights generally ranging from 250,000 to 600,000., herein incorporated by reference.

Another example of a suitable cationic polymer according to the present disclosure is Polyquaternium-16, a polymeric quaternary ammonium salt formed from methylvinylimidazolium chloride and vinylpyrrolidone.

Another example of a suitable cationic polymer according to the present disclosure is Polyquaternium-22, a copolymer of dimethyl diallyl ammonium chloride and acrylic acid.

### Maleic acid and/or a cosmetically acceptable salt thereof

As given in the Final Report on the Safety Assessment of Maleic Acid, Int J of Tox, 26 (Suppl. 2):125-130, 2007, the referenced material hereby incorporated by reference, maleic acid (cis-butenedioic acid; CAS RN 110-16-7) is a dicarboxylic acid that functions as a pH adjuster and fragrance in cosmetics, which presents the molecular formula C₄H₄O₄, the molecular mass 116.07 g/mol and conforms to the following chemical formula

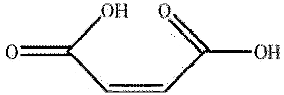

According to the present disclosure, any cosmetically acceptable salt of maleic acid (i.e., maleate) can be used in the compositions described herein. Representative salts include, but are not limited to, salts with cations of ammonium, sodium, potassium, magnesium, calcium, strontium, barium, aluminum, iron, zinc, bismuth and organic amines. Typically, salts of maleic acid according to the present disclosure are salts with cations of ammonium, sodium, potassium and organic amines of maleic acid, such as bis(3-aminopropyl)amine, diglycol bis(3-aminopropyl)amine or poly(ethylene glycol) bis(3-aminopropyl) terminated.

In an embodiment, maleic acid is used in its acid form.

Typically, in the anhydrous composition for bleaching keratin fibers of the present disclosure, the total amount of maleic acid and/or a cosmetically acceptable salt thereof is in the range of about 0.01 to about 10 wt. %, based on the total weight of the composition.

In an embodiment, in the anhydrous composition for bleaching keratin fibers of the disclosure, the total amount of maleic acid and/or a cosmetically acceptable salt thereof is in the range of about 0.01 to about 7.5 wt. %, based on the total weight of the composition.

In another embodiment, in the anhydrous composition for bleaching keratin fibers of the disclosure, the total amount of maleic acid and/or a cosmetically acceptable salt thereof is in the range of about 0.05 to about 5 wt. %, based on the total weight of the composition.

In an embodiment, the anhydrous composition for bleaching keratin fibers comprises, based on the total weight of the composition:
a) about 1-60 wt. % of at least one alkaline agent;
b) about 20-70 wt. % of at least one peroxygenated salt;
c) about 0.01-10 wt. % of at least one cationic polymer; and
d) about 0.01-10 wt. % of maleic acid and/or a cosmetically acceptable salt thereof.

In another embodiment, the anhydrous composition for bleaching keratin fibers comprises, based on the total weight of the composition:
a) about 5-50 wt. % of at least one alkaline agent;
b) about 25-65 wt. % of at least one peroxygenated salt;
c) about 0.01-7.5 wt. % of at least one cationic polymer; and
d) about 0.1-5 wt. % of maleic acid and/or a cosmetically acceptable salt thereof.

In another embodiment, the anhydrous composition for bleaching keratin fibers comprises, based on the total weight of the composition:
a) about 1-60 wt. % of at least one alkaline agent selected from alkali metal or alkaline-earth metal salts of silicates, metasilicates, phosphates, hydrogen phosphates, carbonates, hydrogen carbonates, and mixtures thereof;
b) about 20-70 wt. % of at least one peroxygenated salt selected from ammonium, alkali metal or alkaline-earth metal persulfates, percarbonates, perborates, peroxides, and mixtures thereof;
c) about 0.01-10 wt. % of at least one cationic polymer; and
d) about 0.01-10 wt. % of maleic acid and/or a cosmetically acceptable salt thereof.

In another embodiment, the anhydrous composition for bleaching keratin fibers comprises, based on the total weight of the composition:
a) about 5-50 wt. % of at least one alkaline agent selected from alkali metal or alkaline-earth metal salts of silicates, metasilicates, phosphates, hydrogen phosphates, carbonates, hydrogen carbonates, and mixtures thereof;
b) about 25-65 wt. %, of at least one peroxygenated salt selected from ammonium, alkali metal or alkaline-earth metal persulfates, percarbonates, perborates, peroxides, and mixtures thereof;
c) about 0.01-7.5 wt. % of at least one cationic polymer; and
d) about 0.1-5 wt. %, of maleic acid and/or a cosmetically acceptable salt thereof.

In another embodiment, the anhydrous composition for bleaching keratin fibers comprises, based on the total weight of the composition:
a) about 5-50 wt. % of at least one alkaline agent selected from ammonium chloride, ammonium bromide, ammonium iodide, ammonium sulfate, ammonium nitrate, ammonium phosphates, ammonium nitrate, ammonium dihydrogen phosphate, ammonium hydrogen phosphate, diammonium sodium phosphate, sodium ammonium hydrogen phosphate, ammonium disodium phosphate, ammonium carbonate, ammonium hydrogen carbonate, sodium silicate, potassium silicate, sodium metasilicate, potassium metasilicate, sodium carbonate, potassium carbonate, magnesium carbonate, magnesium carbonate hydroxide (magnesium basic carbonate), and mixtures thereof, and mixtures thereof;
b) about 25-65 wt. %, of at least one peroxygenated salt selected from ammonium persulfate, sodium persulfate, potassium persulfate, magnesium peroxide, and mixtures thereof;
c) about 0.01-7.5 wt. % of at least one cationic polymer selected from Polyquaternium-6, Polyquaternium-7, Polyquaternium-10, Polyquaternium-16, Polyquaternium-22 and mixtures thereof; and
d) about 0.1-5 wt. %, of maleic acid and/or a cosmetically acceptable salt thereof.

In another embodiment, the anhydrous composition for bleaching keratin fibers comprises, based on the total weight of the composition:
a) about 1-60 wt. % of at least one alkaline agent selected from alkali metal or alkaline-earth metal salts of silicates, metasilicates, phosphates, hydrogen phosphates, carbonates, hydrogen carbonates, and mixtures thereof;
b) about 20-70 wt. % of at least one peroxygenated salt selected from ammonium, alkali metal or alkaline-earth metal persulfates, percarbonates, perborates, peroxides, and mixtures thereof;
c) about 0.01-10 wt. % of Polyquaternium-10; and
d) about 0.01-10 wt. % of maleic acid and/or a cosmetically acceptable salt thereof.

The anhydrous bleaching compositions of the present disclosure can optionally further contain anionic, nonionic, cationic, amphoteric or zwitterionic surfactants and mixtures thereof, petroleum hydrocarbons (mineral oils, paraffins and waxes), vegetable fats and oils, fatty acids, fatty alcohols, natural waxes, silicones, granulating adjuvants, binders, mineral fillers, lubricants, opacifiers (such as titanium dioxide), inorganic pigments, dyes, sequestering agents, fragrances or perfumes and nonionic or amphoteric polymers; any additive for facilitating the handling and application of the composition, any additive for improving the storage and efficacy of the compositions, and/or any additive for improving the cosmetic properties of the treated hair of the composition. One of skill in the hair coloring and treating art will be able to determine what combination of additives provide the desired properties to the bleached keratin fibers.

Non-limiting examples of anionic surfactants which can be used, alone or as mixtures, in the context of the present disclosure, are salts (such as alkali metal salts, especially sodium salts, ammonium salts, amine salts, amino alcohol salts or magnesium salts) of the following compounds: alkyl sulphates; alkyl ether sulphates; alkylamidoether sulphates; alkylarylpolyether sulphates; monoglyceride sulphates; alkyl sulphonates; alkyl phosphates; alkylamide sulphonates; alkylaryl sulphonates; alpha-olefin sulphonates; paraffin sulphonates; (C₆-C₂₄) alkyl sulphosuccinates; (C₆-C₂₄) alkyl ether sulphosuccinates, (C₆-C₂₄) alkylamide sulphosuccinates; (C₆-C₂₄) alkyl sulphoacetates; (C₆-C₂₄) acyl sarcosinates and (C₆-C₂₄) acyl glutamates. It is also possible to use (C₆-C₂₄) alkylpolyglycoside carboxylic esters such as alkylglucoside citrates; alkylpolyglycoside tartrate and alkylpolyglycoside sulphosuccinates; alkyl sulphosuccinamates; acyl isethionates and N-acyltaurates; the alkyl or acyl radical of all these various compounds comprising from 12 to 20 carbon atoms, and the aryl radical denoting a phenyl or benzyl group. Among the anionic surfactants non-limiting examples may include fatty acid salts such as oleic, ricinoleic, palmitic (cetylic) and stearic acid salts, coconut oil acid or hydrogenated coconut oil acid; acyl lactylates in which the acyl radical comprises 8 to 20 carbon atoms. Additional non-limiting examples include alkyl D-galactoside uronic acids and salts thereof, polyoxyalkylenated (C₆-C₂₄) alkyl ether carboxylic acids, polyoxyalkylenated (C₆-C₂₄) alkylaryl ether carboxylic acids and polyoxyalkylenated (C₆-C₂₄) alkylamido ether carboxylic acids and salts thereof. It will be noted that the sodium or ammonium salts of alkyl sulphates and alkyl ether sulphates constitute anionic surfactants that are suitable in the context of the present disclosure.

Non-limiting examples of nonionic surfactants in the context of the present disclosure include, but are not limited to, polyethoxylated or polypropoxylated fatty acids, alkylphenols, alpha-diols or alcohols having a fatty chain containing, for example, 8 to 18 carbon atoms, ethylene oxide or propylene oxide groups to range in particular from 2 to 50; copolymers of ethylene oxide and of propylene oxide, condensates of ethylene oxide and of propylene oxide with fatty alcohols; polyethoxylated fatty amides having from 2 to 30 mol of ethylene oxide; polyglycerolated fatty amides containing on average 1 to 5, or 1.5 to 4, glycerol groups; polyethoxylated fatty amines having 2 to 30 mol of ethylene oxide; oxyethylenated fatty acid esters of sorbitan having from 2 to 30 mol of ethylene oxide; fatty acid esters of sucrose; fatty acid esters of polyethylene glycol; alkylpolyglycosides; N-alkylglucamine derivatives; amine oxides such as (C₁₀-C₁₄) alkylamine oxides or N-acylaminopropylmorpholine oxides.

Non-limiting examples of amphoteric or zwitterionic surfactants include, but are not limited to, aliphatic secondary or tertiary amine derivatives in which the aliphatic radical is a linear or branched chain containing 8 to 18 carbon atoms and containing at least one water-soluble anionic group (for example carboxylate, sulphonate, sulphate, phosphate or phosphonate); (C₈-C₂₀) alkylbetaines, sulphobetaines, (C₈-C₂₀) alkylamido (C₁-C₆) alkylbetaines or (C₈-C₂₀) alkylamido (C₁-C₆) alkylsulphobetaines; amine derivatives, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid.

Examples of cationic surfactants include, but are not limited to, polyoxyalkylenated primary, secondary or tertiary fatty amine salts; quaternary ammonium salts; tetraalkylammonium, alkylamidoalkyltrialkylammonium, trialkylbenzylammonium, trialkylhydroxyalkylammonium or alkylpyridinium chlorides or bromides; imidazoline derivatives; or amine oxides of cationic nature.

Non-limiting examples of cationic surfactants are known by their INCI name Quaternium. Non-limiting examples include, but are not limited to: Quaternium-8, Quaternium-14, Quaternium-15, Quaternium-18, Quaternium-22, Quaternium-24, Quaternium-26, Quaternium-27, Quaternium-30, Quaternium-33, Quaternium-43, Quaternium-45, Quaternium-51, Quaternium-53, Quaternium-56, Quaternium-53, Quaternium-60, Quaternium-61, Quaternium-63, Quaternium-70, Quaternium-71, Quaternium-72, Quaternium-73, Quaternium-78, Quaternium-80, Quaternium-81, Quaternium-82, Quaternium-83, Quaternium-84, Quaternium-85, Quaternium-86, Quaternium-87, Quaternium-88, Quaternium-89, Quaternium-90, Quaternium-91, Quaternium-92, Quaternium-93, Quaternium-95, Quaternium-96 and Quaternium-97.

For purposes of this disclosure petroleum hydrocarbons are represented as mineral oils, paraffins and waxes based on petroleum. Examples include, but are not limited to, hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffin waxes (which consist of C₄₀-C₅₅ compounds which contain, in addition to normal hydrocarbons, included in the group areiso-alkanes and naphtenes with long alkyl side-chains, the iso-alkanes forming microcrystals, the microcrystalline paraffin waxes having mean molecular weights comprised between 500 and 800 g/mol, being solids at room temperature, and having melting points comprised between 60°C and 90°C), or mixtures thereof.

For purposes of this disclosure vegetable fats and oils are linear and/or branched esters, linear or branched, saturated and/or unsaturated alkanecarboxylic acids with a chain length of 1 up to 30 carbon atoms, saturated and/or unsaturated alcohols with a chain length of 1 up to 30 carbon atoms; or linear and/or branched esters of aromatic carboxylic acids, or saturated and/or unsaturated alcohols with a chain length of 1 up to 30 carbon atoms. These oils can be selected from the group consisting of for example isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, stearyl palmitate, oleyl palmitate, oleyl oleate, oleyl erucate, erucyl oleate, erucyl erucate, as well as synthetic, semisynthetic and natural mixtures esters, such as jojoba oil (a natural mixture of esters of monounsaturated monocarboxylic acids with a C₁₈-C₂₄ chain with also monounsaturated monoalcohols and with a long C₁₈-C₂₄ chain).

Other examples of vegetable fats and oils include ester oils such as sugar esters or diesters of C₁₂-C₂₄ fatty acids. The term "sugar" means compounds comprising several alcohol functions, with or without an aldehyde or ketone function, and which comprise at least 4 carbon atoms. These sugars may be monosaccharides, oligosaccharides or polysaccharides.
Non-limiting examples of sugars that may be used according to the present disclosure, include sucrose (or saccharose), glucose, galactose, ribose, fucose, maltose, fructose, mannose, arabinose, xylose and lactose, and derivatives thereof, including for example, alkyl derivatives such as methyl derivatives, for instance methylglucose. Non-limiting examples of the sugar esters of fatty acids that may be used according to this disclosure include those from the group comprising esters or mixtures of esters of sugars described above and of linear or branched, saturated or unsaturated C₁₂-C₂₄ fatty acids.
The esters may be chosen from mono-, di-, tri-, tetraesters and polyesters, and mixtures thereof. These esters may be chosen from, for example, but not limited to, oleates, laurates, palmitates, myristates, behenates, cocoates, stearates, linoleates, linolenates, caprates and arachidonates, or mixtures thereof such as oleo-palmitate, oleo-stearate and palmito-stearate mixed esters. It will be noted that the sucrose, glucose or methylglucose monoesters and diesters and for example sucrose, glucose or methylglucose mono- or dioleates, stearates, behenates, oleopalmitates, linoleates, linolenates and oleostearates, constitute sugar esters or diesters of C₁₂-C₂₄ fatty acids that are suitable in the context of the present disclosure. A particular non-limiting example that may be mentioned is methylglucose dioleate.

Other suitable oils of the type of esters of saturated alkane carboxylic acids and alcohols are fatty acid methyl esters, such as C₆-C₂₄ fatty acid methyl esters from animal and vegetable fats and oils such as cotton, safflower, coconut, rapeseed, linseed, palm, palm kernel, sunflower, olein, olive, olive pomace, castor oil, soy, tall oil, etc, possibly totally or partially hydrogenated, as well as purified or synthetic fatty acids such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable vegetable fats and oils according to the present disclosure are fatty acid triglycerides, including for example, linear and/or branched triglycerin esters, saturated and/or unsaturated alkanecarboxylic acids with a chain length of 6 up to 24 carbon atoms, preferably of 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The most simple triglycerides are those constituted by a sole fatty acid.

Fatty acid triglycerides can be chosen, for example, from the group consisting of synthetic, semi-synthetic and natural oils, as for example avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its byproducts such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

For purposes of this disclosure fatty acids include, but are not limited to C₆-C₂₄ fatty acids from vegetable and animal fats and oils, such as those previously described, and cotton, safflower, coconut, rapeseed, linseed, palm, palm kernel, sunflower, olein, olive, olive pomace, castor oil, soy, tall oil, etc, possibly totally or partially hydrogenated, as well as purified or synthetic fatty acids such as caproic acid, caprylic acid, capric acid, lauric acid, myristic acid, palmitic (cetylic) acid, palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or or technical grade mixtures thereof.

For purpose of this disclosure fatty alcohols include, but are not limited to C₆-C₂₄ fatty alcohols from vegetable fats and oils and include cotton, safflower, coconut, rapeseed, linseed, palm, palm kernel, sunflower, olein, olive, olive pomace, castor oil, soy, tall oil, etc, possibly totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures.

Natural waxes according to the present disclosure, include but are not limited to, candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

For purposes of the present disclosure silicones include, cyclic and/or linear silicones, which can be found as monomers generally characterized by structural elements such as: 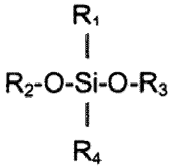 where the silicon atoms can be substituted by alkyl or aryl radicals equal or different, represented here generally by R₁-R₄ groups.

Linear silicones with siloxane units suitable according to the present disclosure are generally characterized by structural elements such as: 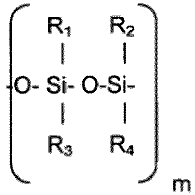 where the silicon atoms can be substituted by alkyl or aryl radicals equal or different, represented here in general by R₁-R₄ groups (meaning the number of different radicals is not necessarily limited to 4), m can take values from 2 to 200.000.

Cyclic silicones suitable according to the present disclosure are generally characterized by structural elements such as: 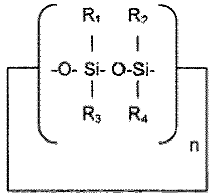 where the silicon atoms can be substituted by alkyl or aryl radicals equal or different, represented here generally by R₁-R₄ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula [(CH₃)₂SiO]x in which x is 3-6, or short chain linear methyl siloxanes having the formula ((CH₃)₂SiO[(CH₃)₂SiO]_{y}Si(CH₃)₃ in which y is 0-5.

Some suitable cyclic methyl siloxanes are hexamethylcyclotrisiloxanes (D3), octamethylcyclotetrasiloxane (D4), decamethylcyclopentasiloxane (D5) (cyclomethicone) and dodecamethylcyclohexasiloxane (D6).

Some suitable short linear methyl siloxane are hexamethyldisiloxane (MM), octamethyltrisiloxane (MDM), decamethyltetrasiloxane (MD2M), dodecamethylpentasiloxane (MD3M), tetradecamethylhexasiloxane (MD4M), and hexadecamethylheptasiloxane (MD5M).

Furthermore, long chain linear siloxanes such as phenyltrimethicone, bis(phenylpropyl)dimethicone, dimethicone, dimethiconol, cyclomethicone (octametilciclotetrasiloxane), hexamethylcyclotrisiloxane, poly(dimethylsiloxane), cetyldimethicone and behenoxy dimethicone are also suitable silicones according to the present disclosure.

For purposes of this disclosure granulating adjuvants, binders, or mineral fillers include, but are not limited to, monosaccharides, disaccharides, starch and starch derivatives, sugar alcohols, calcium phosphate, calcium silicate, powdered cellulose, magnesium silicate, magnesium trisilicate, magnesium carbonate, magnesia, magnesium oxide, sodium lauryl sulfate, magnesium lauryl sulfate, sodium lauryl sulfate starch, silicon dioxide, talc, colloidal silica, colloidal anhydrous silica (colloidal silicon dioxide or fumed silicon dioxide), magnesium stearate, calcium stearate, sodium stearyl fumarate, polyethylene glycol, sodium benzoate, potassium benzoate.

For purposes of this disclosure lubricates are exemplified by light mineral oil, hydrogenated vegetable oils such as castor oil, glycerin monostearate, glyceryl behenate, glyceryl palmitostearate, stearic acid, zinc stearate, and mixtures thereof.

For purposes of this disclosure monosaccharides are colourless, crystalline solids that are freely soluble in water but insoluble in nonpolar solvents. The simplest monosaccharides are the two three-carbon trioses: glyceraldehyde, an aldotriose, and dihydroxyacetone, a ketotriose. Monosaccharides with four, five, six, and seven carbon atoms in their backbones are called, respectively, tetroses, pentoses, hexoses, and heptoses (Lehninger Principles of Biochemistry, 4th edition). Suitable monosaccharides according to the disclosure are D-ribose, D-arabinose, D-xylose, D-glucose (dextrose), D-mannose, D-galactose and D-fructose.

For purposes of this disclosure disaccharides consist of two monosaccharides joined covalently by an O-glycosidic bond, which is formed when a hydroxyl group of one sugar reacts with the anomeric carbon of the other (Lehninger Principles of Biochemistry, 4th edition). Suitable disaccharides according to the disclosure are lactose, include but are not limited to, maltose (a-D-glucopyranosyl-(1 -4)-D-glucopyranose), sucrose (a-D- glucopyranosyl β-D-fructofuranoside) and trehalose (a-D-glucopyranosyl a-D-glucopyranoside).

For purposes of this disclosure a starch (CAS RN 9005-25-8; molecular formula (C₆H₁₀O₅)n where n = 300-1000) is a natural homopolysaccharide, which consists of linear amylose and branched amylopectin, two polysaccharides based on alpha-(D)-glucose. Both polymers are organized in a semicrystalline structure, and in the starch granule, amylopectin forms the crystalline portion. Starch derivatives according to this disclosure refer to partially or fully pre-gelatinized starch (starch that has been chemically and/or mechanically processed to rupture all or part of the starch granules), hydrolized starch, dextrinized starch, alkaline-modified starch (with sodium hydroxide or potassium hydroxide), bleached starch (with hydrogen peroxide), enzyme-treated starch, monostarch phosphate, monostarch phosphate, hydroxypropylated starch, hydroxyethyl starch, octenyl succinic anhydride (OSA) starch, cationic starch (adding positive electrical charge to starch), carboxymethylated starch (with monochloroacetic acid adding negative charge), or mixtures thereof.

For purposes of this disclosure, sugar alcohols are polyalcohols resulting from the reduction of the carbonyl group in a monosaccharide to a hydroxyl group. They are water-soluble solids that occur naturally and are used widely in the food industry as thickeners and sweeteners. Suitable sugar alcohols according to the disclosure are erythritol, isomalt, lactitol, mannitol, maltitol, sorbitol and xylitol.

For purposes of this disclosure opacifiers include, but are not limited to, titanium dioxide (TiO2), latex, styrene/PVP, styrene/acrylamide copolymers, and mixtures thereof.

For purposes of this disclosure inorganic pigments include those having a color index number as listed in the CTFA dictionary, 10th edition, 2004, hereby incorporated by reference.

For purposes of this disclosure dyes include directed dyes selected from anionic, cationic and non-ionic nitro dyes.

Examples of anionic direct dyes include, but are not limited to, Bromophenol Blue, Tetrabromophenol Blue, Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 5, FD&C Yellow No. 6, FD&C Blue 1, Food Black 1, Food Black 2, Disperse Black 9 and Disperse Violet 1 and their alkali metal salts such as sodium, potassium.

For purposes of this disclosure cationic dyes are those available on the market for cosmetic hair coloring applications. Non-limiting examples include: Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31.

For purposes of this disclosure neutral dyes (HC dyes), so called nitro dyes for shading purposes include, but are not limited to, HC Blue No. 2, HC Blue No. 4, HC Blue No. 5, HC Blue No. 6, HC Blue No. 7, HC Blue No. 8, HC Blue No. 9, HC Blue No. 10, HC Blue No. 11, HC Blue No. 12, HC Blue No. 13, HC Blue No. 15, HC Blue No. 18, HC Brown No. 1, HC Brown No. 2, HC Green No. 1, HC Orange No. 1, HC Orange No. 2, HC Orange No. 3, HC Orange No. 5, HC Red BN, HC Red No. 1, HC Red No. 3, HC Red No. 7, HC Red No. 8, HC Red No. 9, HC Red No. 10, HC Red No. 11, HC Red No. 13, HC Red No. 14, HC Red No. 18, HC Red No. 54, HC Violet BS, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

For purposes of this disclosure sequestering agents include, but are not limited to, calcium disodium Ethylenediaminetetraacetate (EDTA), diammonium EDTA, disodium EDTA, dipotassium EDTA, trisodium EDTA, tripotassium EDTA, tetrasodium EDTA, tetrapotassium EDTA, triethanolamine salt of EDTA, HEDTA (Hydroxyethyl Ethylenediamine Triacetic Acid), and mixtures thereof.

For purposes of this disclosure, the term "fragrance" as used herein refers to any substance, natural or synthetic, used to impart an odour to a product.

For purposes of this disclosure, the term "perfume" as used herein refers to any mixture of fragrant essential oils and aroma compounds, fixatives, and solvents used to give the human body, objects, and living spaces a lasting and pleasant smell.

For purposes of this disclosure, non-limiting examples of non-ionic polymers include, but are not limited to:
- nonionic guar gums and modified nonionic guar gums may be for example, but are not limited to, those modified with C₁-C₆ hydroxyalkyl groups;
- biopolysaccharide gums of microbial origin such as scleroglucan gum or xanthan gum;
- gums derived from plant exudates, such as gum arabic, ghatti gum, karaya gum, tragacanth gum, carrageenan, agar and carob gum;
- pectins;
- alginates;
- starches;
- hydroxy (C₁-C₆) alkylcelluloses and carboxy (C₁-C₆) alkylcelluloses;
- celluloses modified with groups comprising at least one fatty chain; non-limiting mention may be made, for example, of hydroxyethylcelluloses modified with groups comprising at least one fatty chain, such as alkyl, arylalkyl or alkylaryl groups, or mixtures thereof, and wherein the alkyl groups are, for example, C₈-C₂₂; or those modified with polyalkylene glycol alkylphenyl ether groups;
- hydroxypropyl guars modified with groups comprising at least one C₈-C₂₂ fatty chain,
- copolymers of vinylpyrrolidone and of hydrophobic monomers comprising a fatty chain, such as vinylpyrrolidone/hexadecene copolymer or vinylpyrrolidone/eicosene copolymer;
- copolymers of C1-C6 alkyl acrylates or methacrylates and of amphiphilic monomers comprising at least one fatty chain;
- copolymers of hydrophilic acrylates or methacrylates and of hydrophobic monomers comprising at least one fatty chain, such as the polyethylene glycol methacrylate/lauryl methacrylate copolymer;
- polymers with an aminoplast ether skeleton comprising at least one fatty chain;
- polyurethane polyethers comprising in their chain both hydrophilic blocks, for example of polyoxyethylenated nature, and hydrophobic blocks that may be aliphatic blocks alone and/or cycloaliphatic and/or aromatic blocks.

For purposes of this disclosure, non-limiting examples of amphoteric polymers include, but are not limited to, amphoteric polysaccharides such as Carboxymethylchitosan or N-[(2'-Hydroxy-2',3'-dicarboxy)ethyl]chitosan; Amphoteric Urethanes; Modified Potato Starch; Methacryloyl Ethyl Betaine/Acrylates Copolymer; Acrylates/Lauryl Acrylate/Stearyl Acrylate/Ethylamine Oxide Methacrylate; and Acrylic acid/(meth)acrylamidopropyl-trimethylarnmonium chloride/stearyl methacrylate terpolymer.

The anhydrous composition for bleaching keratin fibers can be in any form such as dispersion, suspension, powder, dust-free powder, cream, paste, etc. The term "paste" as used herein refers to a composition having a consistency that is intermediate between a solid and a liquid, for example, but not limited to, having a viscosity of greater than about 5 poises at about 25° C, and at a shear rate of about 1 s⁻¹. This viscosity may be measured using a rheometer or a cone-plate viscometer.

The powder composition can be produced with the following process: mixing the powdery ingredients first and subsequently adding lipophilic ingredient(s). The powder can also be prepared by the fluidized bed method. In the fluidized bed method, powder ingredients are mixed in a vessel and made flowing by inletting an air flow which may be heated or carried out at room (ambient) temperature and while the powder mix freely "flowing" lipophilic ingredient and/or mixture with any other liquid component is sprayed from a nozzle mounted above the powder batch.

Typically, when the anhydrous composition for bleaching keratin fibers of the disclosure is in the form of a dust free powder composition, its average particle size is generally below about 1 mm, or below about 500 µm, or less than about 400 µm, or about 25 to about 100 µm, thus ensuring excellent processing capability, i.e. miscibility with an aqueous hydrogen peroxide solution prior to application onto human hair.

The anhydrous composition for bleaching keratin fibers of the present disclosure do not introduce additional steps to the convention bleaching process.

The present disclosure also relates to a process for bleaching keratin fibers, the process comprising the steps of:
i) mixing, before application, an anhydrous composition as defined above, with an aqueous hydrogen peroxide composition;
ii) topically applying the mixture obtained to the keratin fibers to be bleached;
iii) leaving the mixture to stand on the fibers for a period which is sufficient to obtain the bleaching effect; and
iv) removing the mixture from the keratin fibers, for instance by rinsing with water.

In an embodiment, in the process for bleaching keratin fibers as described above, the period of step (iii) ranges from about 5 to about 75 minutes, or from about 10 to about 45 minutes; at a temperature in the range of about 20°C to about 45°C, or from 20°C to 37°C.

In an embodiment, the process for bleaching keratin fibers as described above further comprises the step (v) of:
- topically applying to the keratin fibers a composition comprising at least one conditioning agent, then rinsing with water and optionally followed by washing the keratin fibers with a shampoo, rinsing with water, and optionally drying; or
- washing the keratin fibers with a shampoo, then rinsing with water and optionally followed by topically applying to the keratin fibers a composition comprising at least one conditioning agent, rinsing with water, and optionally drying.

In an embodiment, the process for bleaching keratin fibers comprises the steps of:
i) mixing, before application, an anhydrous composition as defined above, with an aqueous hydrogen peroxide composition;
ii) topically applying the mixture obtained to the keratin fibers to be bleached;
iii) leaving the mixture to stand on the fibers for a period which is sufficient to obtain the bleaching effect;
iv) removing the mixture from the keratin fibers by rinsing with water; and
v) topically applying to the keratin fibers a composition comprising at least one conditioning agent, then rinsing with water and optionally followed by washing the keratin fibers with a shampoo, rinsing with water, and optionally drying.

In another embodiment, the process for bleaching keratin fibers comprises the steps of:
i) mixing, before application, an anhydrous composition as defined above, with an aqueous hydrogen peroxide composition;
ii) topically applying the mixture obtained to the keratin fibers to be bleached;
iii) leaving the mixture to stand on the fibers for a period ranging from about 5 to about 75 minutes, or from about 10 to about 45 minutes; at a temperature in the range of about 20°C to about 45°C, or from about 20°C to about 37°C;
iv) removing the mixture from the keratin fibers by rinsing with water; and
v) topically applying to the keratin fibers a composition comprising at least one conditioning agent, then rinsing with water and optionally followed by washing the keratin fibers with a shampoo, rinsing with water, and optionally drying.

In another embodiment, the process for bleaching keratin fibers comprises the steps of:
i) mixing, before application, an anhydrous composition as defined above, with an aqueous hydrogen peroxide composition;
ii) topically applying the mixture obtained to the keratin fibers to be bleached;
iii) leaving the mixture to stand on the fibers for a period which is sufficient to obtain the bleaching effect; and
iv) removing the mixture from the keratin fibers by rinsing with water;
v) washing the keratin fibers with a shampoo, then rinsing with water and optionally followed by topically applying to the keratin fibers a composition comprising at least one conditioning agent, rinsing with water, and optionally drying.

In another embodiment, the process for bleaching keratin fibers comprises the steps of:
i) mixing, before application, an anhydrous composition as defined above, with an aqueous hydrogen peroxide composition;
ii) topically applying the mixture obtained to the keratin fibers to be bleached;
iii) leaving the mixture to stand on the fibers for a period ranging from about 5 to about 75 minutes, or from about 10 to about 45 minutes; at a temperature in the range of about 20°C to about 45°C, or from about 20°C to about 37°C; and
iv) removing the mixture from the keratin fibers by rinsing with water;
v) washing the keratin fibers with a shampoo, then rinsing with water and optionally followed by topically applying to the keratin fibers a composition comprising at least one conditioning agent, rinsing with water, and optionally drying.

Typically, the conditioning agent is selected from cationic polymers, cationic surfactants, petroleum hydrocarbons (mineral oils, paraffins and waxes), vegetable fats and oils, fatty acids, fatty alcohols, natural waxes or silicones as described above.

In an embodiment, the composition comprising at least one conditioning agent of step (v) further comprises maleic acid and/or a cosmetically acceptable salt thereof.

In an embodiment, the shampoo of step (v) further comprises maleic acid and/or a cosmetically acceptable salt thereof.

The disclosure also provides the use of an anhydrous composition as described above for the preparation of a ready-to-use human keratin fiber bleaching composition. For this, the anhydrous composition can be mixed with about 0.5 to about 10 equivalents by weight of an aqueous hydrogen peroxide composition, for example, a solution, an emulsion, a gel or a cream. Typically, the aqueous hydrogen peroxide composition comprises about 2 to about 12 % by weight of hydrogen peroxide.
It should be noted that the mixing ratio of the anhydrous composition of the disclosure and the aqueous hydrogen peroxide composition is very much dependent on the level of bleaching effect targeted, i.e. the level of highlighting and/or bleaching and darkness of hair before bleaching, and can be adjusted accordingly by hair dressers. The above mentioned ratios are general and in case somewhat different mixing ratios are needed simply because of reaching higher bleaching level than usual levels, such mixing ratio should still be understood being within the scope of the present disclosure.

The aqueous hydrogen peroxide composition may further comprise those substances customarily found in oxidizing compositions such as chelating agents, such as Ethylenediaminetetraacetate (EDTA), and/or its salts. Typically, the pH of this aqueous hydrogen peroxide composition is in the range of about 2 to about 7, or in the range of about 2.5 to about 6, or in the range of about 2.5 to about 5.

Typically, the pH of the ready-to-use keratin fiber bleaching composition, mixture of the anhydrous composition of the disclosure and the aqueous hydrogen peroxide composition, is in the range of about 8 to about 12, or in the range of about 9 to about 11.

The following examples are given in order to provide a person skilled in the art with a sufficiently clear and complete explanation of the present disclosure, but should not be considered as limiting of the essential aspects of its subject, as set out in the preceding portions of this description.

### EXAMPLES

### Example 1

### Example 1.1. - Preparation of the bleaching compositions

Four powder bleaching compositions were prepared by combining the following components together and by mixing until homogeneity in a suitable mixer: Potassium Persulfate; Sodium Silicate; Ammonium Persulfate; Sodium Metasilicate; Sodium Stearate; Magnesium Carbonate; Guar Gum; Maleic Acid; Sodium Lauryl Sulfate; Tetrasodium EDTA; Xanthan Gum; Paraffinum Liquidum; Parfum (Fragrance) and Ultramarines, and are differentiated as follows:
- the first powder bleaching composition does not comprise any additional ingredient. (P1);
- the second powder bleaching composition additionally contains maleic acid (P2);
- the third powder bleaching composition additionally contains Polyquaternium-10 (P3); and
- the fourth powder bleaching composition contains maleic acid and Polyquaternium-10 (P4).

The powder bleaching composition P1 to P3 are comparative while the powder bleaching composition P4 is a composition according to the present disclosure.

Each powder bleaching composition (P1 to P4) is mixed in a non-metallic bowl at a ratio 1:2 by weight with a commercially available 9% by weight aqueous hydrogen peroxide composition.

The weight percentage of each ingredient in the thus obtained ready-to-use bleaching compositions (R1 to R4) are indicated in Table 1. It has to be remarked that the wt.% of water was adjusted in the ready-to-use composition R1 to R3 to keep constant the concentration of alkaline agents and peroxygenated salts in all ready-to-use compositions.

**Table 1. - Ready-to-use bleaching compositions for keratin fibers (wt. % based on the total weight of the composition).**

| **Ingredients** | **Bleaching compositions** | | | |
|---|---|---|---|---|
| | **R1** | **R2** | **R3** | **R4** |
| Potassium Persulfate | 17.88 | 17.88 | 17.88 | 17.88 |
| Sodium Silicate | 7.89 | 7.89 | 7.89 | 7.89 |
| Ammonium Persulfate | 1.60 | 1.60 | 1.60 | 1.60 |
| Sodium Metasilicate | 1.55 | 1.55 | 1.55 | 1.55 |
| Sodium Stearate | 1.25 | 1.25 | 1.25 | 1.25 |
| Magnesium Carbonate | 0.78 | 0.78 | 0.78 | 0.78 |
| Guar Gum | 0.67 | 0.67 | 0.67 | 0.67 |
| Maleic Acid | --- | 0.67 | --- | 0.67 |
| Polyquaternium-10 | --- | --- | 0.67 | 0.67 |
| Sodium Lauryl Sulfate | 0.29 | 0.29 | 0.29 | 0.29 |
| Tetrasodium EDTA | 0.28 | 0.28 | 0.28 | 0.28 |
| Xanthan Gum | 0.14 | 0.14 | 0.14 | 0.14 |
| Paraffinum Liquidum | 0.10 | 0.10 | 0.10 | 0.10 |
| Parfum (Fragrance) | 0.09 | 0.09 | 0.09 | 0.09 |
| Ultramarines (Cl 77007) | 0.16 | 0.16 | 0.16 | 0.16 |
| Aqua (Water) (Eau) | 58.62 | 57.95 | 57.95 | 57.28 |
| Hydrogen Peroxide | 6.00 | 6.00 | 6.00 | 6.00 |
| Emulsifiers, pH adjusters and thickeners from aqueous hydrogen peroxide composition | Up to 100 | Up to 100 | Up to 100 | Up to 100 |

### Example 1.2. - Application of the bleaching compositions

The ready-to-use compositions R1 to R4 are applied to swatches of virgin medium brown Caucasian hair. 24 hours prior to the bleaching treatment, the swatches are washed with a shampoo and rinsed with deionized water. This process is repeated three times to replicate hair that has been over processed by bleaching.
- First application:
   Each ready-to-use composition R1 to R4 are brushed to both sides of different dry swatches of hair in order to thoroughly and uniformly coat the swatches. The swatches are wrapped in aluminum foil and are allowed to stand in an oven at 37°C for a period of 30 minutes. Then, the swatches are rinsed for 5-10 minutes with tap water.
      After that, the swatches are shampooed for 1 minute and rinsed for 1 minute with tap water at 38°C. The swatches are completely blown dry.
- Second application:
   The first application was repeated to the same dried swatches.
      In this case the exposure time in the oven is 15 minutes. The swatches are removed from the oven and rinsed for 5-10 minutes with tap water, shampoed for 1 minute and rinsed for 1 minute with tap water at 38°C. Then, the swatches are completely blow dried and are thus ready for the third application.
- Third application:
   The first application was repeated to the same dried swatches.
      In this case the exposure time in the oven is 15 minutes. The swatches are rinsed for 5-10 minutes with tap water, shampooed for 1 minute and rinsed for 1 minute with tap water at 38°C. Then, the swatches are completely blow dried.

### Example 1.3. - Evaluation of the bleaching compositions

The evaluation is performed by expert professional cosmetologists using a blind test. The bleaching compositions are evaluated by professional cosmetologists different from the ones that applied the bleaching compositions on the swatches of hair. Four expert professional cosmetologists assessed care and efficacy performance attributes using salon grading scores ranging from 1 (not acceptable) to 5 (very good) as indicated in Table 2.

**Table 2 - Scoring scale definitions of the parameters evaluated**

| **Parameter** | **Definition** | **Score** |
|---|---|---|
| Hair strength | Ability to avoid hair breakage | 1=Not acceptable; 2=Deficient; 3=Good; 4=Very good; 5=Excellent |
| Touch feel | Feeling of the hair paying special attention from mid to ends | 1=Not acceptable; 2=Deficient; 3=Good; 4=Very good; 5=Excellent |
| Combability | Try to comb the hair with a standard comb and evaluate the combing easiness | 1=Not acceptable; 2=Deficient; 3=Good; 4=Very good; 5=Excellent |

Hair strength (strength/elasticity): hair fiber has an elastic characteristic, and it may undergo moderate stretching either wet or dry. Stretching is a hair attribute under the action of a distal force (length) and the thread returns to the original status, when this force stops again. When dry, the hair may stretch 20-30% of its length; and, in contact with water, this may reach up to 50%. A damaged hair is fragile (hair breaks under pressure) and non-elastic (it does not returns to the original status under a force) Measurement was made by taking 5-10 hair fibers, which are stretched from the roots to the ends 3 times approximately one third of their original length.
It is scored from 1: Not acceptable (broken hair) to 5: Excellent (completely elastic recovery)

Touch feel: healthy hair has a smooth touch feel because the external layer (cuticle) is sealed and cells are aligned forming a smooth surface.

A damaged hair has a non-aligned cuticle with holes on them, producing a rough surface.

Measurement is made by taking a small tuft of hair and passing the fingers from the roots to the ends.
It is scored from 1: Not acceptable (very rough touch) to 5: Excellent (completely smooth and slippery).

Combability: may be defined as the subjective perception of the easy or difficult way for combing the hair. It is directly directed with the forces with are opposite to the action of combing the hair. Healthy hair has a smooth and lubricated surface; there is a minimum interaction between hair fibers and non-friction, the force need to comb is low. Damaged hair has a rough surface and a missing lipid layer. The friction between fibers increase and also the force needed to comb the hair.
It is measured with a standard comb pass it through from the roots to the ends.
It is scored from 1: Not acceptable (comb sticks) to 5: Excellent (very easy to comb).

Based on the experience in evaluating products, a score difference of one point between two compositions indicates a property that is discernable by consumers. The results represent the mean values of the scores for each parameter.
The assessed parameters are determined after the third application of the bleaching compositions to the hair swatches immediately after rinsing the ready-to-use compositions, i.e. in wet hair; and after shampooing, rinsing and drying the hair swatches, i.e. dry hair.

**Table 3. - Results of the evaluation of the hair attributes after bleaching**

| **Hair attribute** | **Score** | | | |
|---|---|---|---|---|
| | **R1** | **R2** | **R3** | **R4** |
| After third bleaching application - wet hair | | | | |
| Dry hair strength | 1.0 | 2.5 | 1.3 | 3.3 |
| Dry touch feel | 2.0 | 2.8 | 2.3 | 3.8 |
| Dry combability | 2.0 | 2.8 | 2.3 | 3.8 |

| After third bleaching application - dry hair | | | | |
|---|---|---|---|---|
| Dry hair strength | 1.3 | 3.0 | 1.5 | 4.0 |
| Dry touch feel | 1.8 | 3.3 | 1.8 | 4.5 |
| Dry combability | 1.8 | 2.8 | 1.3 | 4.3 |

Table 3 shows the results of the evaluation of the swatches of hair treated with the ready-to-use compositions obtained with the four ready-to-use bleaching compositions described in Table 1. This table shows a synergistic effect in the properties of hair strength (ability to avoid hair breakage), touch feeling (feeling of the hair), and combability in the swatches of hair treated with a ready-to-use composition obtained with the powder bleaching composition P4 containing a cationic polymer such as Polyquaternium-10 and maleic acid versus the comparative compositions P1 to P3.

The improvement in the hair attributes is observed in both wet and dry hair. In addition, this improvement in hair attributes (reduction in hair damage) has been observed after a repeated bleaching process (i.e. three subsequent bleaches), where the hair was more damaged. Furthermore,

From the experimental results, it can be concluded that the anhydrous compositions for bleaching keratin fibers of the disclosure not only provide a reduced degree of hair damage but also do not introduce additional steps to a conventional bleaching process.

## Claims

1. An anhydrous composition for bleaching keratin fibers comprising at least one cationic polymer and maleic acid and/or a cosmetically acceptable salt thereof.

2. The composition according to claim 1, wherein the at least one cationic polymer is selected from Polyquaternium-6, Polyquaternium-7, Polyquaternium-10, Polyquaternium-16, Polyquaternium-22 and mixtures thereof.

3. The composition according to claim 1 or 2, wherein the maleic acid is present in acid form.

4. The composition according to anyone of claims 1 to 3, wherein the total amount of the at least one cationic polymer is in the range of about 0.01 to about 10 wt. %, based on the total weight of the composition.

5. The composition according to anyone of claims 1 to 4, wherein the total amount of maleic acid and/or a cosmetically acceptable salt thereof is in the range of about 0.01 to about 10 wt. %, based on the total weight of the composition.

6. The composition according to anyone of claims 1 to 5, wherein the composition further comprises anionic, nonionic, cationic, amphoteric or zwitterionic surfactants and mixtures thereof, petroleum hydrocarbons (mineral oils, paraffins and waxes), vegetable fats and oils, fatty acids, fatty alcohols, natural waxes, silicones, granulating adjuvants, binders, mineral fillers, lubricants, opacifiers, inorganic pigments, dyes, sequestering agents, fragrances or perfumes and nonionic or amphoteric polymers.

7. A process for bleaching keratin fibers comprising the steps of:
i) mixing, before application, an anhydrous composition as defined in any one of claims 1 to 6, with an aqueous hydrogen peroxide composition;
ii) topically applying the mixture obtained to the keratin fibers to be bleached;
iii) leaving the mixture to stand on the fibers for a period which is sufficient to obtain the bleaching effect; and
iv) removing the mixture from the keratin fibers.

8. The process for bleaching keratin fibers according to claim 7, further comprising the step (v) of
- topically applying to the keratin fibers a composition comprising at least one conditioning agent, then rinsing with water and optionally followed by washing the keratin fibers with a shampoo, rinsing with water, and optionally drying; or
- washing the keratin fibers with a shampoo, then rinsing with water and optionally followed by topically applying to the keratin fibers a composition comprising at least one conditioning agent, rinsing with water, and optionally drying.

9. A multi-compartment device for bleaching keratin fibers comprising at least two compartments, wherein one compartment comprises an anhydrous composition as defined in any one of claims 1 to 6; and a second compartment comprising an aqueous hydrogen peroxide composition.
